Europäisches Patentamt

European Patent Office

Office européen des brevets

(11) Publication number: **0 341 361**
A2

# EUROPEAN PATENT APPLICATION

(21) Application number: 88308664.7

(51) Int. Cl.⁴: **A61M 16/04**

(22) Date of filing: 19.09.88

Pursuant to rule 85 (2)(3) EPC the priority 08.09.87 GB 8721051 has been validly claimed.

(30) Priority: 08.09.87 GB 8721051

(43) Date of publication of application: 15.11.89 Bulletin 89/46

(84) Designated Contracting States: AT BE CH DE ES FR GB GR IT LI LU NL SE

(71) Applicant: WARNE SURGICAL PRODUCTS LTD.
Portadown Road Lurgan
County Armagh Northern Ireland(GB)

(72) Inventor: Mehta, Sukhdev Dr Burnside
11 Reedley Drive Burnley
Lancashire DN10 2QZ(GB)

(74) Representative: Allen, Oliver John Richard et al
Lloyd Wise, Tregear & Co. Norman House
105-109 Strand
London, WC2R 0AE(GB)

(54) Endotracheal tube or catheter.

(57) An endotracheal or tracheostomy tube or catheter is described which is provided with a loose-fitting cover over the mouth of the tube and means to remove the cover once the tube has been correctly inserted into for example the trachea or nasal passage. The cover prevents mucus entering the mouth of the tube. Moreover when the tube is provided at the inserted end with an outer sleeve or balloon which may be inflated with air or liquid so that it acts as a gas seal and cushions the internal tissues of the patient, the cover also acts to protect the inflated balloon.

EP 0 341 361 A2

## ENDOTRACHEAL TUBE OR CATHETER

The invention relates to an endotracheal tube or catheter for use as an artificial airway or to a tracheostomy tube.

A standard endotracheal tube comprises a length of flexible tubing to be inserted into the nasal passage or trachea. The tube may be provided, towards the inserted end, with an outer sleeve or balloon which may be inflated with air or liquid once the tube has been positioned correctly in the trachea or nasal passage.

This balloon then acts to provide a gas seal, as a cushion to protect the internal tissues from traumatic damage and also to hold the tube in place.

A disadvantage with such catheters is that during insertion there is a tendency for the inserted (or "patient") end or mouth of the tube to become blocked with saliva and mucus so that air cannot efficiently reach the lungs through the tube.

A endotracheal or tracheostomy tube or catheter in accordance with the invention has a loose-fitting cover over the mouth of the tube and means to remove the cover once the tube has been correctly inserted.

Preferably the cover is arranged when in its operative position to extend from the mouth of the tube which it covers to a position in which it also covers the inflated balloon (when provided) so that it can protect the balloon, and is preferably of a soft, flexible plastic material.

The cover removing means is preferably a narrow plastic tube or flexible rod which is of substantially smaller diameter than the endotracheal tube, and which, at one end, is firmly attached to the inside of the cover. This narrow tube or rod extends through the central bore of the endotracheal tube and emerges through its insertion or "machine" end. The narrow tube or rod may protrude beyond the end of the endotracheal tube for approximately 10 cms.

When an endotracheal tube in accordance with the invention is inserted into the nose or trachea, the cover prevents mucus entering the mouth of the tube and acts to protect the balloon. Once the internal balloon is inflated and the endotracheal tube is held in place, the protruding end of the narrow tube may be gently pulled to remove the cover which preferably turns inside out and passes from the mouth of the endotracheal tube, down through the central bore and out through the external. "machine" end. As the contaminated surface of the cover is located away from the wall due to the turning of the cover "inside out", no fouling of the inner wall occurs and a clear passageway is provided through which air may be supplied to the patient.

The invention will now be described by way of example with reference to the accompanying drawings in which:-

Fig. 1 is a longitudinal section through an example of an endotracheal tube in accordance with the invention with the cover in its operative position, and,

Fig. 2 is a view corresponding to that of Fig. 1 but showing the balloon inflated and the cover removed.

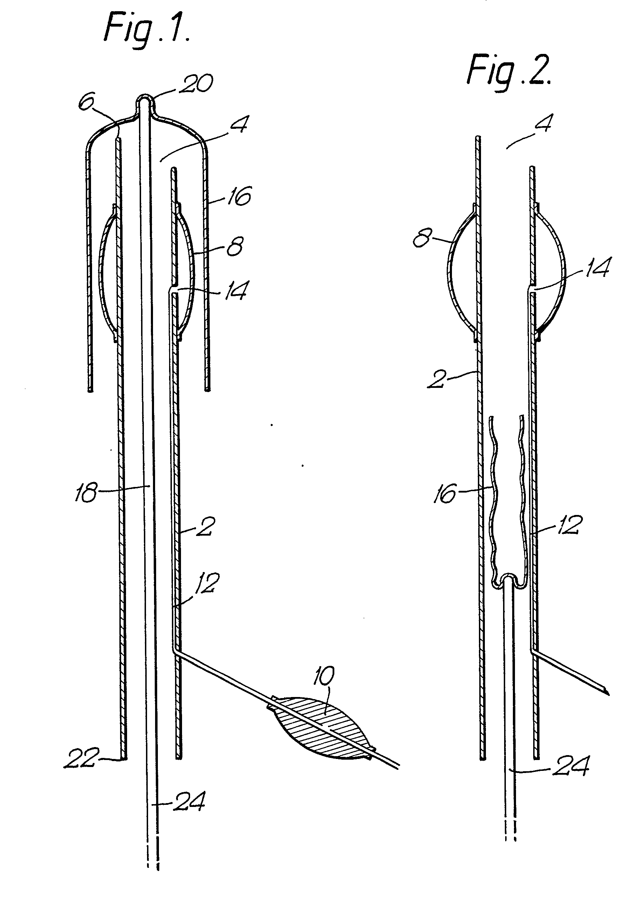

Referring to Fig. 1 the endotracheal tube comprises a length of flexible tube 2, the end 4 to be inserted being cut obliquely to form a mouth and having rounded edges 6.

A sleeve or balloon 8 is fitted around the tube 2 just below the mouth 4. Air or liquid is supplied to inflate the balloon 8 from an external pre-inflated balloon 10 via capillary tube 12 and outlet 14.

A loose-fitting cover 16 of a soft flexible material covers the mouth 4 of the tube 2 and extends down over the outside of the tube to a point below the balloon 8. A narrow tube 18 of substantially smaller diameter than the endotracheal tube 2, is firmly attached to the inside of the cover at 20. The tube 18 extends down through the central bore of the tube 2 and protrudes from the outer or machine end 22 of the tube at 24, a sufficient distance for it to be firmly gripped.

As the tube is inserted mucus and other matter which might otherwise block the mouth of the tube is retained on the outer surface of the cover. The endotracheal tube 2 is inserted into the nose or trachea and correctly positioned. The balloon 8 is then inflated by pumping the external balloon 10. The narrow tube 24 which protrudes from the external end 22 of the endotracheal tube 2 is then pulled gently thus turning the flexible cover 16 inside out and dragging it through the interior of tube 2 and out through the machine end 22 (see Fig. 2).

During movement of the cover 16 through tube 2 the surface thereof which is contaminated by mucus and the like faces away from the inner wall of the tube and thus after removal a clear airway to the patients lungs is provided.

## Claims

1. An endotracheal or tracheostomy tube or catheter having a loose-fitting cover over the mouth of the tube and means to remove the cover once the tube has been inserted.

2. An endotracheal or tracheostomy tube or catheter as claimed in Claim 1 wherein the cover is formed from a soft flexible plastic material.

3. An endotracheal or tracheostomy tube or catheter as claimed in either Claim 1 or 2 wherein the tube is provided with an outer sleeve towards the end of the tube which is inserted, the outer sleeve or balloon being capable of being inflated with air or liquid once the tube has been positioned correctly.

4. An endotracheal or tracheostomy tube or catheter wherein the cover is arranged in its operative position to not only cover the mouth of the tube but also to cover the balloon both on insertion and when inflated.

5. An endotracheal or tracheostomy tube or catheter as claimed in any preceding claim wherein the cover removing means is so arranged that when operated it turns the cover inside out and pulls it through the tube away from the inserted end and out through the opposite end.

6. An endotracheal or tracheostomy tube or catheter as claimed in any of the preceding claims wherein the cover removing means is a narrow plastic tube or plastic rod of substantially smaller diameter than the endotracheal or tracheostomy tube or catheter and which, at one end, is firmly attached to the inside of the cover.

7. An endotracheal or tracheostomy tube or catheter as claimed in claim 6 wherein the narrow tube or rod extends through the central bore of the endotracheal or tracheostomy tube or catheter and protrudes out of the opposite end to that which is inserted.

8. An endotracheal or tracheostomy tube as claimed in claim 7 wherein the plastic tube or rod protrudes beyond the end of the endotracheal or tracheostomy tube or catheter by approximately 10 cms.

9. An endotracheal or tracheostomy tube or catheter as hereinbefore described with reference to Figures 1 and 2.

*Fig .1.*

*Fig .2.*